# FASCICULE DE BREVET EUROPEEN

(11) **EP 2 093 231 B1**
(45) Date de publication et mention de la délivrance du brevet: **07.10.2015**
(21) Numéro de dépôt: 08170734.1
(22) Date de dépôt: 04.12.2008
(51) Int. Cl.: C07H 15/04, A23L 1/236

(54) **Cristaux de maltitol de forme parallélépipèderectangulaire**
Rechteckiger parallelepipedförmiger Maltitolkrystalle
Cristals of maltitol in rectangular parallelepipedal form

(30) Priorité: 12.12.2007 FR 0759793
(43) Date de publication de la demande: 26.08.2009
(73) Titulaire: Roquette Frères, 62136 Lestrem (FR)
(72) Inventeur: Barata, Manuel, 62920, Gonnehem (FR); Duflot, Pierrick, 62136, La Couture (FR); Bensouissi Abdelfattah, 51100, Reims (FR); Mathlouthi, Mohamed, 51390, Coulommes-La-Montagne (FR)
(74) Mandataire: Cabinet Plasseraud

(56) Documents cités:
- EP-A- 0 905 138
- US-A- 4 408 041
- US-A- 5 580 601

## Description

La présente invention concerne des cristaux de maltitol de forme particulière et des compositions cristallines les contenant. Elle a trait également à un procédé particulier d'obtention de ces cristaux et compositions.

Le maltitol (1,4-O-α-D-glucopyranosyl-D-glucitol) est un polyol obtenu par hydrogénation du maltose.

L'absence d'extrémité réductrice dans la molécule de maltitol lui confère une stabilité élevée tant sur le plan thermique que sur le plan chimique.

Moins calorique que le saccharose, il possède des propriétés organoleptiques voisines de ce sucre. Non cariogène, il est alors utilisé dans bon nombre d'applications tant alimentaires que pharmaceutiques.

Pendant très longtemps, le maltitol n'a été commercialisé que sous la forme de sirops de faible richesse, ou sous la forme de poudres amorphes et impures.

Ce n'est qu'au début des années 1980, que la société HAYASHIBARA décrivait pour la première fois dans son brevet US 4.408.041 la production de cristaux de maltitol anhydre.

Auparavant, ce polyol avait toujours été considéré comme un produit non cristallisable.

Ce postulat erroné trouve en réalité son origine dans le fait que la cristallisation du maltitol à partir d'une solution sursaturée n'est pas aussi facile à contrôler que dans le cas d'autres polyols comme le mannitol ou l'érythritol par exemple.

Les techniques dites de "massé" d'une part et de cristallisation dans l'eau d'autre part, sont aujourd'hui quasiment les seuls procédés employés industriellement.

Les produits ainsi obtenus ont cependant une cristallinité très variable, et ne conviennent pas tous particulièrement bien à certaines applications comme celles du chewing-gum ou du chocolat.

Ces produits cristallisés ne sont pas non plus totalement satisfaisants lorsque par exemple on souhaite utiliser du maltitol pour remplacer le saccharose ou le lactose dans les formes sèches pharmaceutiques telles que les gélules, les médicaments du type poudres à dissoudre, les comprimés et les préparations nutritives pulvérulentes à diluer.

C'est également le cas lorsque l'on souhaite réaliser le même genre de substitution dans les aliments sucrés tels que les boissons en poudre, les entremets, les préparations pour gâteaux ou les poudres chocolatées ou vanillées pour petit déjeuner.

On constate pour ces applications particulières, notamment pour les poudres pseudo-cristallines de maltitol obtenues par la technique de "massé", et à un degré moindre pour les poudres cristallines de maltitol obtenues par cristallisation dans l'eau, que celles-ci présentent un ou plusieurs défauts comme en particulier ceux de :
- s'écouler difficilement,
- d'être sujettes à une prise en masse ou à un mottage,
- de ne se dissoudre que très lentement dans l'eau,
- d'être de mauvais excipients pour compression ou
- de ne pas satisfaire aux critères d'identification et de pureté imposés par différentes pharmacopées.

Les spécialistes du domaine de la cristallisation des polyols se sont donc attachés à mettre au point des compositions de maltitol n'ayant pas les défauts d'écoulement, de mottage, de dissolution, ou de compression que présentent les poudres de maltitol connues.

Certes, on aurait pu penser que le besoin identifié puisse être satisfait par d'autres polyols. Or, on constate qu'il n'en est rien car aucun d'entre eux ne possède des caractéristiques de solubilité, d'hygroscopicité, de saveur sucrée et de fusion aussi proches du saccharose que le maltitol.

Cependant, comme on le constate aisément en analysant les différentes contributions relevées dans l'état de la technique, très peu de travaux entrepris se sont préoccupés de l'influence que pouvait avoir la forme des cristaux de maltitol dans la résolution des défauts cités ci-avant.

Le corollaire est que peu de travaux se sont attachés à déterminer quels pouvaient être les conditions de cristallisation permettant de favoriser l'obtention de forme particulière des cristaux de maltitol.

Dans le brevet US 4.408.041, par exemple, pour parvenir à cristalliser le maltitol, HAYASHIBARA recommande seulement de partir d'un sirop de maltitol présentant une teneur élevée en maltitol, et par voie de conséquence, présentant un faible contenu en polyols de plus haut DP (DP3 et DP supérieur ou égal à 4).

On constate alors qu'il se contente de présenter les cristaux ainsi obtenus comme « sans couleur, sans odeur et transparents ».

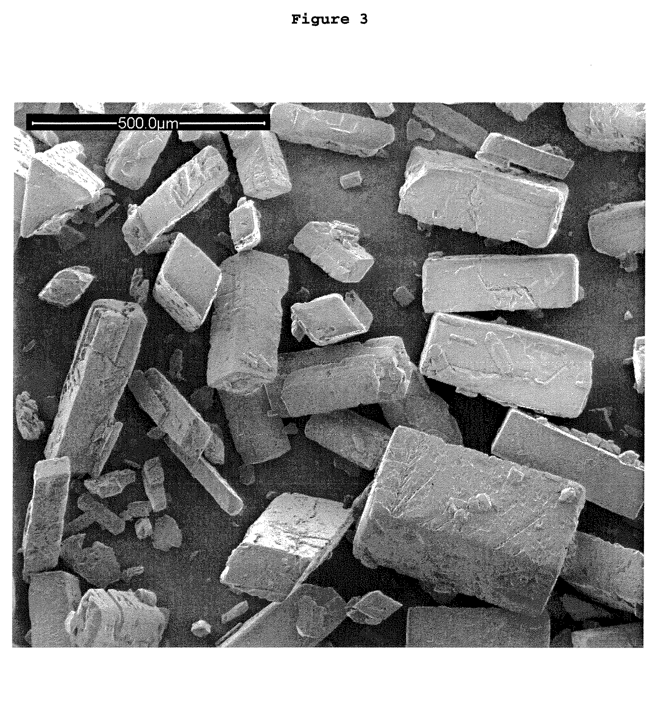

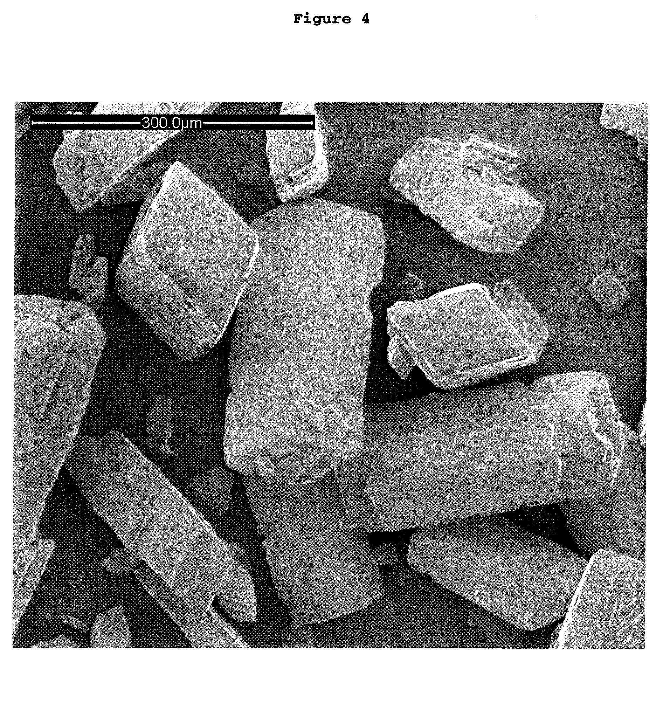

La description des cristaux obtenus est faite par le biais de photos de microscopie électronique (la figure 3 est une vue d'ensemble desdits cristaux (vue dite à x 150), et la figure 4 est prise à un grossissement plus important (x 600)), mais on constate que HAYASHIBARA ne les commente pas.

La figure 4 permet à l'homme du métier de constater que les cristaux de maltitol obtenus sont de forme « parallélépipédique - plutôt cubique ».

En 1982, dans son article scientifique publié dans Carbohydrate Research relatif à l'analyse par cristallographie RX du maltitol, on pourrait s'attendre à ce que la société HAYASHIBARA décrive un peu mieux ces cristaux.

Or, d'un mot, OHNO et al. les décrivent comme des cristaux « incolores et ressemblant à des « prismes » ("prism-like") » et n'associent pas cette forme cristalline à une quelconque propriété fonctionnelle du maltitol.

En fait, le cristal observé dans cet article est un « macro-cristal » qui a été spécialement fabriqué de manière à permettre la détermination des paramètres de maille cristalline par diffraction X.

Le cristal est un « prisme cubique », puisque les auteurs lui donnent les dimensions de 0,3 x 0,3 x 0,3 mm.

En 1989, la société Demanderesse, dans son brevet US 4.846.139 décrivait elle-même également un procédé de préparation de maltitol cristallin.

La cristallisation était faite dans de l'eau. La discussion ne porte cependant pas sur les cristaux, et aucune forme particulière n'est remarquée.

Dans le brevet US 5.583.215 délivré à TOWA, il est décrit un mélange cristallin solide contenant du maltitol. La solution de maltitol de départ qui est cristallisée contient 80 à 98 % de maltitol, 0,5 à 15 % de sorbitol et 1,5 à 10 % de maltotriitol et de DP ≥ 3.

La cristallisation est effectuée par extrusion.

La seule mention de l' « allure » du mélange de cristaux obtenus est celle d'une structure « *crushed and relatively tight* », mais aucune forme particulière des cristaux n'est remarquée ni discutée.

Il faut attendre une autre contribution de la société Demanderesse pour que l'influence de la taille et de la forme des cristaux soient reconnues importantes dans certaines applications alimentaires, et notamment dans la confection par exemple de confiseries au maltitol.

Dans le brevet US 5.580.601, la société Demanderesse décrit en effet l'utilisation de cristaux de xylitol et de maltitol pour des confiseries grainées.

Pour le maltitol destiné à cette application, quatre qualités de maltitol de l'époque sont comparées :
∘ du MALTISORB^{®} P90 et du MALTISORB^{®} P200, commercialisés par la société Demanderesse,
∘ du maltitol commercialisé par la société TOWA sous le nom de marque AMALTY^{®} MR100,
∘ du maltitol fabriqué en suivant l'enseignement de l'exemple 2 du brevet US 4.408.041 de HAYASHIBARA.

La Société Demanderesse, dans ce brevet US 5.580.601, remarque que ces quatre variétés de maltitol se présentaient sous deux formes cristallines différentes : une forme en aiguilles et une forme parallélépipédique plutôt cubique (celle-là même présentée par HAYASHIBARA dans son brevet US 4.408.041).

Il est vérifié en effet par la société Demanderesse dans son brevet US 5.580.601 que lors du processus de cristallisation utilisé conformément à l'exemple 2 du brevet US 4.408.041 de HAYASHIBARA, le sirop de maltitol permet d'obtenir des cristaux de forme « parallélépipédique - plutôt cubique ».

Par ailleurs, il est également précisé que la poudre finement broyée du MALTISORB^{®} P90 en aiguilles redonne des cristaux parallélépipédiques plutôt cubiques.

Dans ce brevet US 5.580.601 la société Demanderesse discute enfin l'influence de la taille et de la forme des cristaux de maltitol sur la qualité texturale et la stabilité au cours du temps des confiseries grainées ainsi préparées.

On retient ainsi que :
- il n'est possible de préparer des confiseries grainées au maltitol qui soient d'excellente qualité texturale et particulièrement stables au cours du temps, que si celles-ci contenaient de 50 à 93 % de maltitol par rapport à la charge édulcorante totale, et que si le maltitol, présent au moins partiellement à l'état cristallisé au sein de la confiserie, l'était essentiellement sous forme de cristaux de taille inférieure à 60 microns, de préférence inférieure à 40 microns et plus préférentiellement encore inférieure à 20 microns,
- si les cristaux contenus dans la confiserie ont une forme en aiguilles, c'est à dire par exemple ont une longueur d'environ 120 microns et une section d'environ 35 microns, alors la confiserie a tendance à évoluer rapidement et à durcir de façon excessive. Ceci n'est pas le cas lorsqu'elle contient des cristaux, de préférence de forme parallélépipédique plutôt cubique, de taille inférieure à 60 microns. Dans ce cas, les confiseries grainées sont stables pendant plusieurs mois. Les cristaux ont alors une pureté en maltitol élevée, dépassant 95 % et le plus souvent 98 %, voire 99 %.

Dans son brevet EP 735.042, la société Demanderesse décrit une nouvelle composition cristalline du maltitol. Cette fois, la cristallisation est effectuée par atomisation.

Les cristaux obtenus sont décrits comme étant de structure essentiellement poreuse et alvéolée, sous forme de particules essentiellement sphériques, dépourvues d'arrêtes vives et composées d'une multitude de microparticules cristallines agglomérées entre elles.

La structure observée se différencie de façon nette de celle d'un maltitol cristallisé dans l'eau et d'un maltitol extrudé, constitués de particules parallélépipédiques plutôt cubiques.

Dans un autre brevet, le brevet EP 741.140, la société TOWA décrit un procédé de préparation de maltitol cristallin, et d'un mélange solide cristallin de maltitol cristallisé. Ici, la cristallisation est effectuée dans l'eau.

Cependant, aucune indication n'est fournie quant à la forme des cristaux.

Il faut attendre le dépôt par la société Demanderesse du brevet EP 905.138 pour qu'une autre avancée majeure soit réalisée.

Dans ce brevet, la société Demanderesse a mis au point des compositions de maltitol n'ayant pas les défauts d'écoulement, de mottage, de dissolution, ou de compression que présentent les poudres de maltitol connues.

C'est en travaillant sur la mise au point de ces compositions de maltitol que la Demanderesse a pu isoler deux formes particulières de cristaux de maltitol, autres que la forme en aiguilles et la forme parallélépipède plutôt cubique : la forme bipyramidale et la forme prismatique.

La société Demanderesse expliquait alors l'existence de ces deux formes de cristaux de maltitol par la teneur en maltotriitol du sirop de maltitol destiné à être cristallisé.

La société Demanderesse a constaté en effet qu'en contrôlant la teneur en maltotriitol d'un sirop de maltitol, il était possible d'orienter la forme des cristaux de maltitol vers l'une ou l'autre des formes ou vers un mélange des deux formes, lorsque ce sirop de maltitol est soumis à une étape de cristallisation.

La société Demanderesse faisait remarquer fort justement dans son brevet EP 905.138 que les formes de cristallisation (bipyramidales ou prismatiques) ont nécessairement des conséquences importantes tant au niveau de la fabrication que des applications.

Ainsi, une masse semi-cristallisée de maltitol, comprenant un certain pourcentage de cristaux prismatiques est plus visqueuse qu'une masse comprenant le même pourcentage de cristaux bipyramidaux, toutes choses étant égales par ailleurs, et cela notamment lorsque les cristaux sont de taille importante.

C'est ainsi que pour préparer du maltitol atomisé, il est préférable de retenir des suspensions très pauvres en maltotriitol et comprenant de plus des cristaux bipyramidaux plutôt que prismatiques pour éviter un empâtement.

Par ailleurs, l'utilisation de cristaux de maltitol bipyramidaux s'avère intéressante pour la production de chocolat (masse plus liée avant raffinage), de chewing-gums (possibilité de conserver une texture souple avec une quantité élevée de maltitol pulvérulent), de formes sèches pharmaceutiques (meilleur écoulement) etc ...

A contrario, une forme prismatique est plus compressible et permet un empâtement (prise en masse) à faible teneur en cristaux, recherché parfois (pâtes à mâcher, centres de chewing-gum à dragéifier).

De tout ce qui précède, il résulte qu'il apparaît important de choisir des tailles et des formes tout à fait particulières des cristaux de maltitol, lorsque l'on souhaite l'employer de manière spécifique dans un domaine applicatif donné.

Quatre formes cristallines sont donc répertoriées :
- la forme parallélépipède plutôt cubique,
- la forme prismatique,
- la forme bipyramidale, et
- la forme en aiguilles.

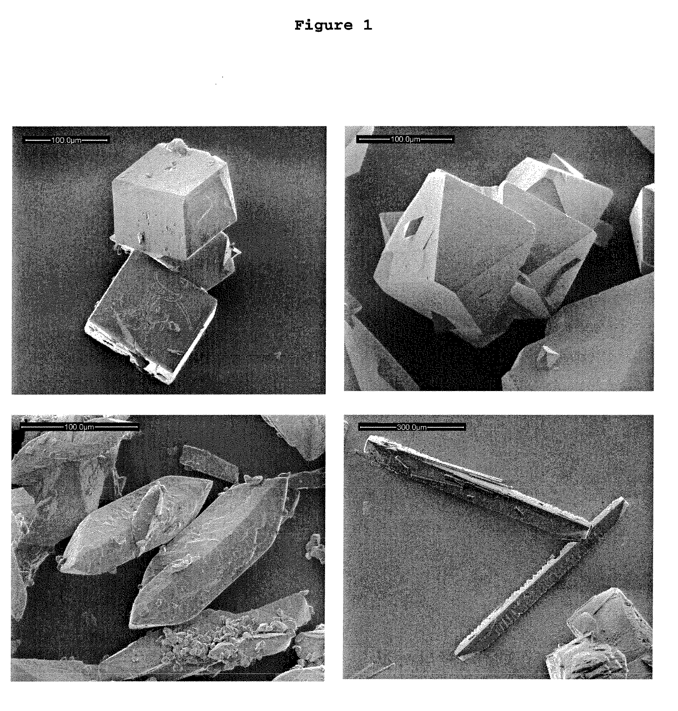

La figure 1 illustre ces quatre formes cristallines (respectivement 1.1, 1.2, 1.3 et 1.4) (photos réalisées par microscopie électronique à balayage dans les conditions telles que définies ci-après).

C'est en poursuivant ses recherches sur la cristallisation du maltitol, que la société Demanderesse a trouvé, contre toute attente, que le maltitol pouvait cristalliser selon une cinquième forme cristalline totalement différente des formes précédemment décrites, ouvrant par là même d'autres voies d'applications au maltitol cristallisé : la forme parallélépipède rectangulaire.

Au sens de l'invention, on entend par forme « parallélépipède rectangulaire » un polyèdre dont au moins quatre des six faces sont des rectangles.

Les observations ont été réalisées à l'aide d'un microscope électronique à balayage FEI modèle QUANTA 200 FEG.

Les cristaux ont été observés sous une tension de 1 keV.

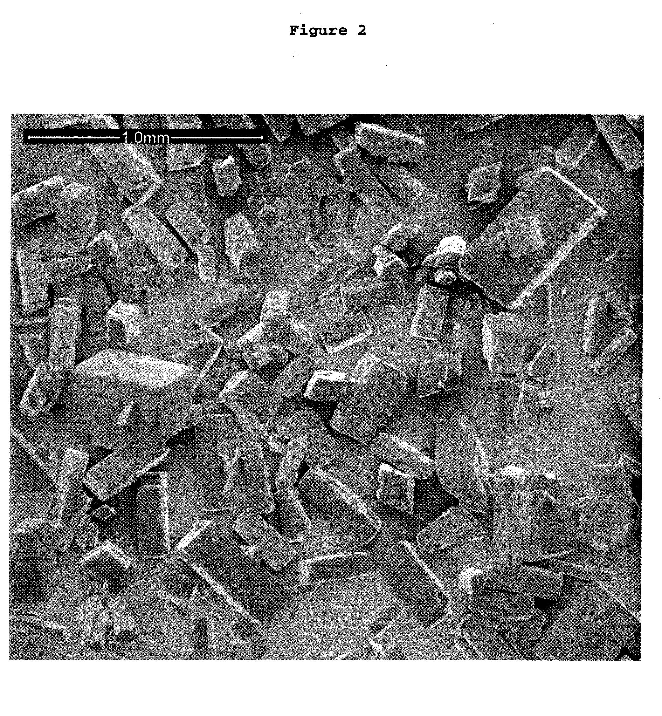

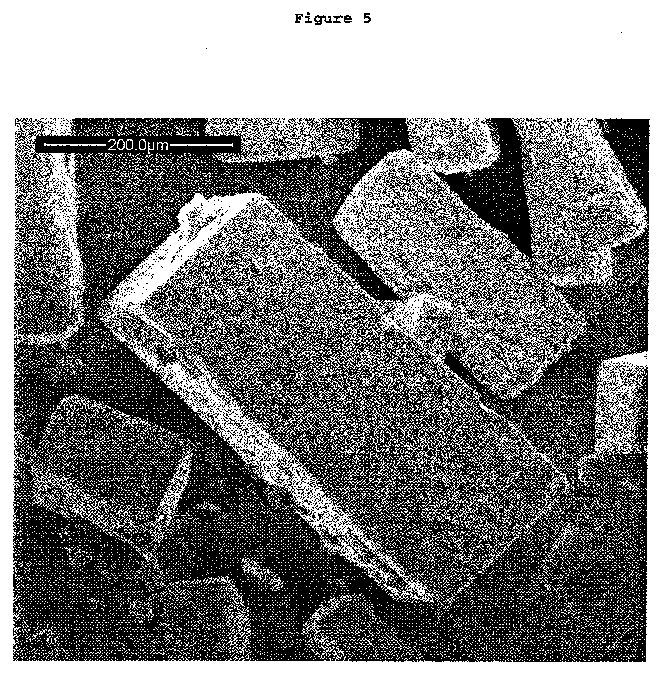

L'invention concerne donc tout d'abord les cristaux de forme parallélépipède rectangulaire illustrés aux figures 2 à 9.

Ces cristaux ne sont pas agglomérés ou organisés en petits paquets agglutinés mais sont au contraire bien dissociés et individualisés les uns par rapport aux autres.

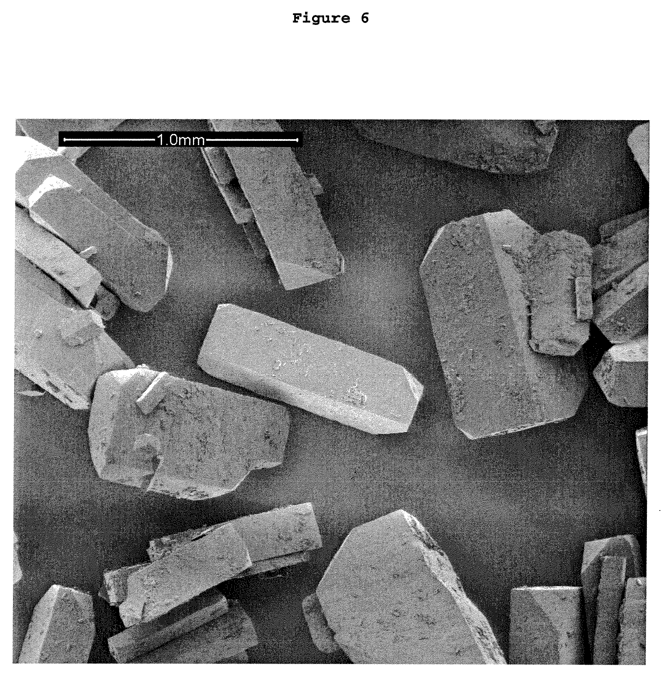

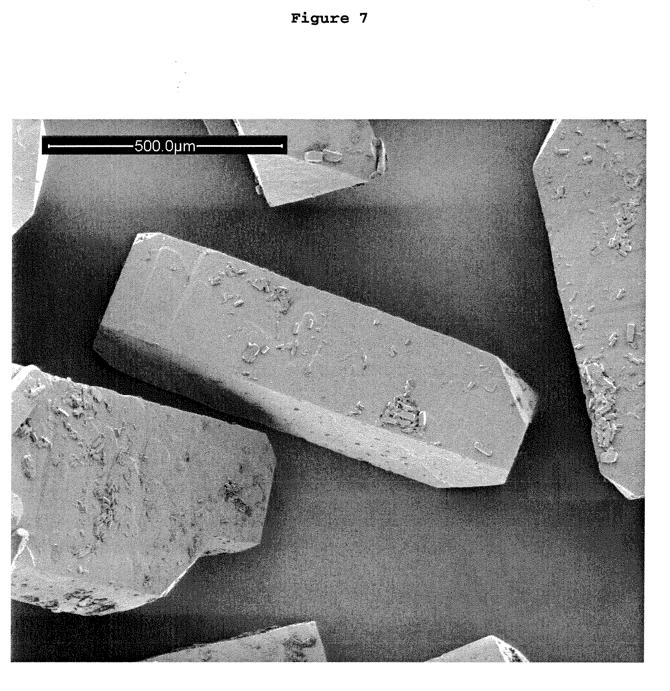

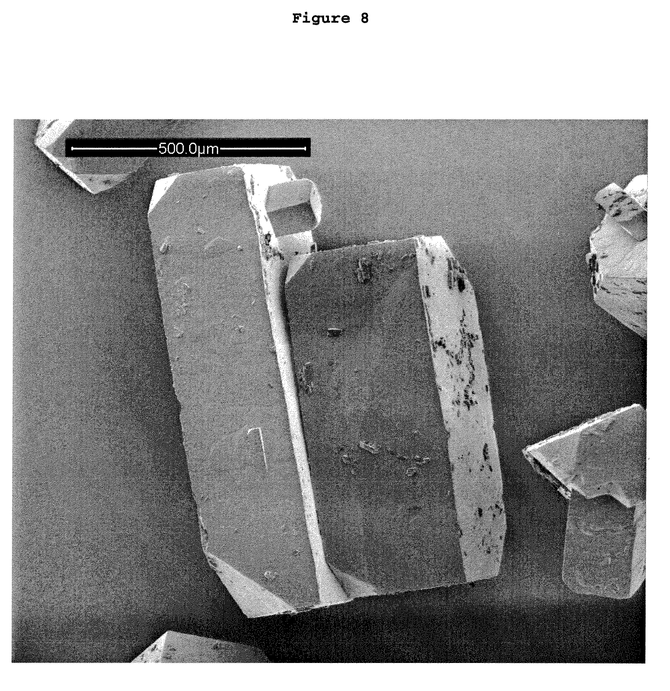

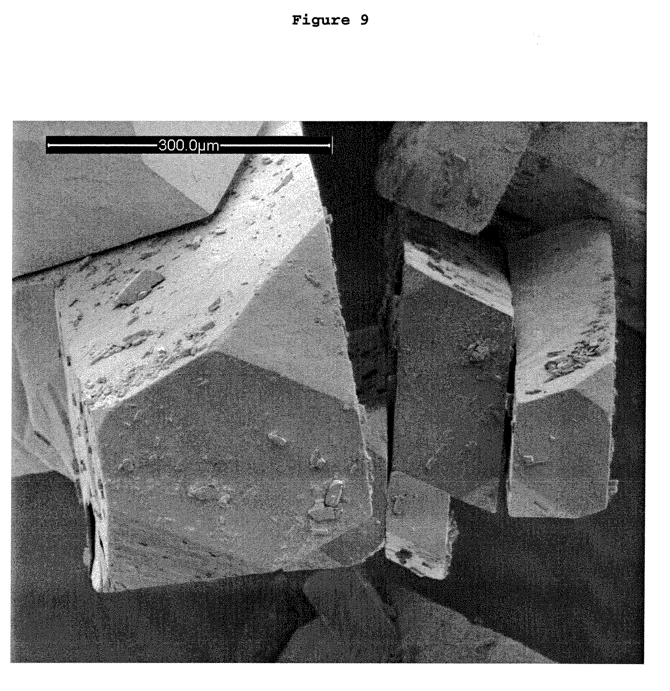

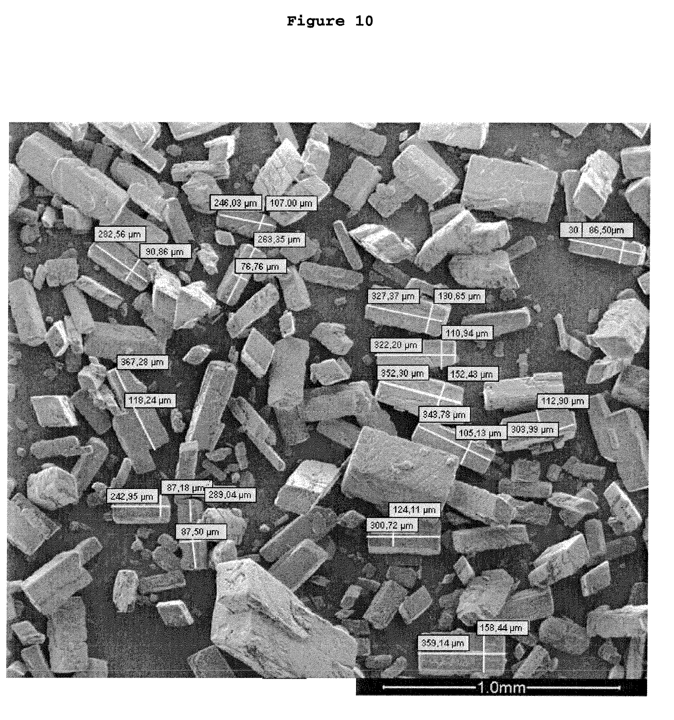

Les photographies sont captées sur le microscope avec un grossissement :
- de 50 fois (figure 2), de 100 fois (figure 3), de 200 fois (figure 4 et 5) pour une observation d'un premier lot de cristaux conformes à l'invention,
- de 50 fois (figures 6 et 10), de 100 fois (figure 7 et 8) et de 200 fois (figure 9) pour une observation d'un second lot de cristaux conformes à l'invention.

Les figures illustrent bien le fait que les cristaux de maltitol sont de forme parallélépipède rectangulaire, au sens de l'invention.

Si l'on considère le schéma ci-dessous, en prenant comme base la plus grande surface (définie par les points AEHD ou BFGC), on constate en effet que les faces latérales (ABFE et DCGH) sont des parallélogrammes dont l'angle α que forme l'arête latérale avec le plan de base est variable, et où les autres faces sont des parallélogrammes rectangles.

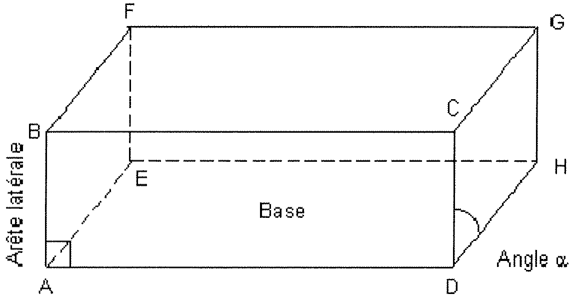

Dans ce cas le parallélépipède rectangulaire est défini par 4 dimensions, la longueur (ici AD), la largeur (AE) et la hauteur (AB) ainsi que la valeur de l'angle α.

Selon l'invention, l'angle α varie de 90° à 50°.

Deux populations de cristaux parallélépipèdes rectangulaires sont alors observées, populations pouvant coexister en toutes proportions au sein d'une même composition au sein de l'invention :
- des cristaux de maltitol, caractérisés par le fait que les deux plus petites faces du parallélépipède rectangulaire (les faces délimitées ABFE et DCGH sur le schéma ci-dessus) sont des parallélogrammes dont les côtés sont sensiblement égaux.

En ce cas, la forme des cristaux est celle d'un polyèdre dont quatre des six faces sont des rectangles, les deux autres pouvant être des losanges (la valeur de l'angle α pouvant être supérieure ou égale à 50° et inférieure à 90°, de préférence comprise entre 60° et 70°) ou des carrés (l'angle α valant 90°).
- d'autres cristaux de maltitol, caractérisés par le fait que les deux plus petites faces sont des parallélogrammes dont les côtés sont égaux deux à deux (l'angle α valant 90°).

En ce cas, la forme des cristaux est celle d'un polyèdre dont quatre faces sont des rectangles, les deux autres sont également des parallélogrammes rectangles. Selon cette éventualité, l'homme du métier obtient une forme géométrique désignée comme celle du parallélépipède rectangle.

Les figures 6 à 9 montrent des cristaux de maltitol selon l'invention, caractérisés par le fait que les deux plus petites faces du parallélépipède rectangulaire sont des parallélogrammes dont au moins un angle présente une troncature.

Les cristaux de maltitol selon l'invention sont caractérisés par le fait que le rapport longueur (dimension AD sur le schéma ci-dessus) sur largeur (dimension AE sur le schéma ci-dessus) du parallélépipède rectangulaire est compris entre 1,8 et 5,3, de préférence compris entre 2,3 et 3,7.

Cette valeur a été déterminée par la mesure du rapport des tailles d'une trentaine de cristaux individualisés, telle que présentée dans la figure 10 (sur laquelle sont matérialisées et quantifiées les dimensions de quelques cristaux).

Les valeurs ont été obtenues par la détermination de la largeur et de la longueur des faces rectangulaires de chacun des parallélépipèdes rectangulaires observés.

Les cristaux de maltitol selon l'invention sont également caractérisés par le fait que la longueur du parallélépipède rectangulaire (dimension AD sur le schéma ci-dessus) est comprise entre 50 et 750 µm, de préférence comprise entre 97 et 280 µm.

Pour permettre l'identification des formes cristallines, on utilise une technique de diffraction des rayons X en réflexion de la poudre de cristaux de maltitol selon le test A suivant.

Le test A consiste à déterminer des intensités relatives de raies de diffraction. Les spectres de diffraction sont obtenus avec un diffractomètre des rayons X pour analyse de poudre, équipé d'une source cobalt. L'analyse est effectuée en réflexion avec un porte-échantillon tournant. L'échantillon est compacté manuellement dans le porte-échantillon. L'analyse est effectuée, en continu, de 4° à 34°. Les intensités de raies sont mesurées et normalisées par rapport à la raie la plus intense du spectre de diffraction.

Les cristaux de maltitol selon l'invention sont alors caractérisés par le fait que leur profil de diffraction X, déterminé selon le test A, présente pour la raie à 26,51°, une intensité de 100 %, et pour la raie à 16, 07°, une intensité égale à 0 %.

Comme il sera exemplifié dans l'exemple comparatif donné ci-après, les cristaux de maltitol conformes à l'invention présentent un profil tout à fait unique par rapport aux quatre autres formes cristallines connues du maltitol.

Le maltitol de forme bipyramidale et celui de forme parallélépipède cubique donnent en effet :
- une intensité comprise entre 0 et 0,15 % pour la raie à 16,07°,
   et se caractérisent par l'intensité de leur raie à 26,51° :
- entre 54,46 et 81,76 % pour la forme parallélépipède cubique,
- entre 60,1 et 73,7 % pour la forme bipyramidale.

Quant au maltitol de forme prismatique et celui en aiguilles, ils donnent pour la raie à 26,51° une intensité de 100 % mais pour la raie à 16,07 °C :
- une intensité comprise entre 0,14 et 0,26 % pour la forme prismatique et
- une intensité comprise entre 0,33 et 0,42 % pour la forme en aiguilles.

L'invention concerne encore une composition cristalline de maltitol, caractérisée par le fait qu'elle est constituée en tout ou partie de cristaux de forme parallélépipède rectangulaire.

Cette composition cristalline de maltitol est notamment caractérisée en ce qu'en fait la fraction cristalline du maltitol est constituée d'au moins 50 % en poids de cristaux de forme parallélépipède rectangulaire, de préférence de 70 à 100 % en poids, plus préférentiellement encore de 95 à 100 % en poids.

Pour préparer le sirop de maltitol qui permet, après cristallisation, d'obtenir les formes cristallines du maltitol conformes à l'invention, on met en oeuvre le procédé décrit ci-dessous ou un procédé équivalent.

La première étape du procédé est en soi connue. Elle consiste à liquéfier un lait d'amidon dont l'origine botanique peut être quelconque : il peut provenir du blé, du maïs ou de la pomme de terre par exemple.

Ce lait d'amidon ou de fécule est additionné d'acide dans le cas d'une liquéfaction dite acide, ou d'une α-amylase dans le cas d'une liquéfaction enzymatique.

Dans le procédé conforme à l'invention, on préfère effectuer une hydrolyse ménagée du lait d'amidon de façon à obtenir un lait d'amidon liquéfié à faible taux de transformation. Ainsi, les conditions de température, de pH, de taux d'enzyme et de calcium, connues de l'homme du métier, sont déterminées de manière telle qu'elles permettent d'obtenir un DE (Dextrose Equivalent) inférieur à 10, de préférence inférieur à 6, et plus particulièrement inférieur à 4.

De préférence, l'étape de liquéfaction est conduite en deux sous-étapes, la première consistant à chauffer, pendant quelques minutes et à une température comprise entre 105 et 108°C, le lait d'amidon en présence d'une α-amylase (type TERMAMYL® 120L commercialisée par la société NOVOZYMES) et d'un activateur à base de calcium, la seconde consistant à chauffer le lait d'amidon ainsi traité à une température comprise entre 95 et 100°C pendant une à deux heures.

Une fois l'étape de liquéfaction terminée, dans les conditions de teneur en matières sèches, de pH, de taux d'enzyme et de calcium bien connues de l'homme du métier, on procède à l'inhibition de l'α-amylase. Cette inhibition de l'α-amylase peut se faire de préférence par voie thermique, en procédant en sortie de liquéfaction à un choc thermique de quelques secondes à une température supérieure ou égale à 130°C.

On effectue ensuite l'étape de saccharification. Lors de cette étape, on soumet tout d'abord le lait d'amidon liquéfié à l'action d'une α-amylase maltogénique, telle que celle commercialisée par la société NOVO, sous le nom MALTOGENASE®. Lors de cette première étape de saccharification, l'α-amylase maltogénique peut être ajoutée en une seule fois ou en plusieurs fois.

On poursuit ensuite, après avoir laissé agir l'α-amylase maltogénique, la saccharification du lait d'amidon liquéfié au moyen d'une β-amylase telle que celle commercialisée par la société GENENCOR sous la dénomination SPEZYME® BBA 1500.

Lors de ces étapes, il convient d'associer aux enzymes ayant une activité maltogénique (α-amylase maltogénique et β-amylase) une enzyme hydrolysant spécifiquement les liaisons alpha -1,6 de l'amidon. Cet ajout d'une enzyme débranchante permet d'une part d'accélérer les réactions d'hydrolyse sans simultanément accélérer les réactions de réversion et, d'autre part, de réduire la quantité d'oligosaccharides hautement branchés résistant normalement à l'action des enzymes maltogéniques.

Cet ajout d'enzyme débranchante peut se faire au moment de l'ajout de l'α-amylase maltogénique ou au moment de l'ajout de la β-amylase.

Cette enzyme débranchante est choisie dans le groupe constitué par les pullulanases et les isoamylases.

La pullulanase est, par exemple, celle commercialisée par la société ABM sous la dénomination PULLUZYME®.

L'isoamylase est, par exemple, celle commercialisée par la société HAYASHIBARA.

L'étape de saccharification peut être conduite également totalement ou partiellement en présence d'α-amylase fongique.

En fin de saccharification, il est possible d'ajouter un peu d'α-amylase, ce qui améliore généralement les étapes subséquentes de filtration. Les quantités et les conditions d'action des différentes enzymes mises en oeuvre dans les étapes de liquéfaction et de saccharification du lait d'amidon sont généralement celles qui sont recommandées pour l'hydrolyse de l'amidon et sont bien connues de l'homme du métier.

On effectue la saccharification jusqu'à ce que l'hydrolysat de maltose contienne au moins 85 % en poids de maltose (sec / sec).

L'hydrolysat ainsi saccharifié est ensuite filtré sur filtre à précouche ou par microfiltration sur membranes, puis déminéralisé.

L'hydrolysat de maltose ainsi obtenu peut alors être facilement hydrogéné catalytiquement.

L'hydrogénation d'un tel hydrolysat s'effectue conformément aux règles de l'art qui conduisent par exemple à la production de sorbitol à partir du glucose.

On peut utiliser pour cette étape aussi bien des catalyseurs à base de ruthénium que des catalyseurs au nickel de RANEY. On préfère cependant utiliser des catalyseurs au nickel de RANEY qui sont moins onéreux.

Dans la pratique, on utilise de 1 à 10 % en poids de catalyseur par rapport à la matière sèche de l'hydrolysat soumis à l'hydrogénation. L'hydrogénation s'effectue de préférence sur un hydrolysat dont la matière sèche est comprise entre 15 et 50 %, dans la pratique plutôt comprise entre 30 et 45 %, sous une pression d'hydrogène comprise entre 20 et 200 bars. Elle peut être effectuée de manière continue ou discontinue.

Lorsque l'on opère de manière discontinue, la pression d'hydrogène utilisée est généralement comprise entre 30 et 60 bars et la température à laquelle se déroule l'hydrogénation est comprise entre 100 et 150°C. On veille aussi à maintenir le pH du milieu d'hydrogénation par l'addition de soude ou de carbonate de soude par exemple, mais sans dépasser un pH de 9,0. Cette manière de faire permet d'éviter l'apparition de produits de cracking ou d'isomérisation.

On arrête la réaction lorsque la teneur du milieu réactionnel en sucres réducteurs est devenue inférieure à 1 %, de préférence encore inférieure à 0,5 % et plus particulièrement inférieure à 0,1 % (sec/sec).

Après refroidissement du milieu réactionnel, on élimine le catalyseur par filtration et on déminéralise le sirop de maltitol ainsi obtenu sur des résines cationiques et anioniques. A ce stade, les sirops contiennent au moins 80 % de maltitol (sec/sec).

Pour obtenir les formes cristallines du maltitol conforme à l'invention, la société Demanderesse a trouvé qu'il faut conduire la cristallisation du sirop de maltitol dans des conditions particulières.

Il est connu en toute généralité de l'homme du métier que la forme finale d'un cristal dépend largement des conditions de cristallisation.

Les impuretés modifient également la forme (ou habitus) du cristal, telles les formes prismatiques et bipyramidales du cristal de maltitol qui sont obtenues en présence de différentes concentrations de maltotriitol, comme il a été décrit plus haut (conformément à l'enseignement du brevet EP 905.138).

La société Demanderesse a ainsi trouvé, et c'est ce qui constitue une des caractéristiques essentielles du procédé conforme à l'invention, que l'on peut orienter la forme cristalline du maltitol vers la forme parallélépipède rectangulaire en réalisant la cristallisation du sirop de maltitol en présence d'aldéhydes et de composés dicarbonylés condensés.

Ces derniers sont obtenus notamment par un traitement thermique prononcé, i.e. à plus de 100°C, de solutions glucidiques de type saccharose, fructose, sucres invertis, sirops de glucose.

La société demanderesse a trouvé que la quantité d'impuretés devait être inférieure à 5%, de préférence dans un intervalle de 1,5 à 4,5 %, et plus préférentiellement dans un intervalle de 2,5 à 3,5 %.

Un mode préférentiel de réalisation du procédé conforme à l'invention consiste en ce que l'on :
a) prépare un premier sirop de maltitol d'une matière sèche au moins égale à 60 % en poids et présentant une teneur en maltitol au moins égale à 85 % en poids,
b) introduit dans ce sirop de maltitol un sucre choisi dans le groupe constitué par le saccharose, le fructose, les sucres invertis, les sirops de glucose, seuls ou en mélange
c) chauffe ce premier sirop de maltitol de manière à générer des impuretés constituées d'aldéhydes et de composés dicarbonylés condensés,
c) prépare un deuxième sirop de maltitol d'une matière sèche au moins égale à 65 % en poids et présentant une teneur en maltitol au moins égale à 90 % en poids,
e) incorpore ce deuxième sirop de maltitol dans le premier sirop de maltitol de manière à obtenir un sirop de maltitol d'une matière sèche au moins égale à 70 % en poids et présentant une teneur en maltitol au moins égale à 80 % en poids,
f) concentre le sirop de maltitol ainsi obtenu à une matière sèche au moins égale à 80 %,
g) cristallise par évaporation et/ou par refroidissement le sirop de maltitol ainsi concentré,
h) récupère les cristaux ainsi obtenus.

L'invention sera mieux comprise à la lecture des exemples qui suivent et qui se veulent illustratif et non limitatif.

### Exemple 1

On prépare un sirop de maltitol dans lequel on introduit du fructose de manière à ce qu'il présente la composition suivante (sirop A), sa matière sèche étant par ailleurs de 60 % :
- 88 % sur sec de maltitol,
- 4 % sur sec de sorbitol,
- 2 % sur sec de DP ≥ 3 hydrogénés,
- 1 % de produit de cracking
- 5 % sur sec de fructose.

On chauffe à pression atmosphérique à 120°c durant 2 heures.

On obtient alors un sirop de maltitol (sirop B) présentant, une matière sèche de 75 % :
- 37 % sur sec de maltitol,
- 19 % sur sec de sorbitol,
- 37 % sur sec de DP ≥ 3,
- 7 % de produit de cracking renfermant essentiellement des aldéhydes et des composés dicarbonylés condensés issus de la caramélisation.

On prépare également un sirop de maltitol (sirop C), présentant, pour une matière sèche de 67 % :
- 93 % sur sec de maltitol,
- 3 % sur sec de sorbitol,
- 2 % sur sec de DP ≥ 3,
- 2 % de produit de cracking

L'incorporation du sirop C dans le sirop B dans une proportion de l'ordre de 3,7 pour 1 conduit à obtenir un sirop de maltitol (sirop D), présentant quant à lui, pour une matière sèche de 70 % :
- 81,1 % sur sec de maltitol,
- 6,4 % sur sec de sorbitol,
- 9,4 % sur sec de DP ≥ 3,
- 3,1 % de produit de cracking

Ce sirop D est concentré à 84 % de matière sèche par évacuation des buées.

On amène ce sirop concentré à 70°C, puis on amorce avec 0.05% sec de cristaux de maltitol broyés à 35 µm.

On refroidit par augmentation du vide dans un intervalle de 28 à 11 kPa de manière à réguler la température de 70°C à 50°C en 2 heures avec recyclage des buées.

Une seconde cristallisation est effectuée par refroidissement, afin d'augmenter le rendement de cristallisation et d'augmenter la taille des cristaux, en transférant la totalité de la masse cristallisée obtenue à l'étape précédente dans un réacteur double enveloppe puis en refroidissant de 50 à 45°c en 5 heures.

On obtient alors des eaux mères présentant, pour une matière sèche de 72 % :
- 68 % sur sec de maltitol,
- 12 % sur sec de sorbitol,
- 17 % sur sec de DP ≥ 3,
- 3 % de produit de cracking

La composition cristalline obtenue contient quant à elle 97,6 % sur sec de maltitol, 0,2 % sur sec de sorbitol et 2,2 % sur sec de DP ≥ 3.

Les formes cristallines sont toutes majoritairement de type parallélépipède rectangulaire telles qu'illustrées par les figures 2 à 5.

### Exemple 2

La cristallisation du sirop D de l'exemple 1 (84 % de matière sèche) est réalisée dans un évapocristallisoir refroidit par évaporation adiabatique.

Les buées y sont condensées en continu et réincorporées.

Le temps de séjour est de 5 heures, ce qui permet de baisser la température du sirop D, initialement fixée à 70°C, telle que décrite dans l'exemple 1, à une température de 50°C.

La massecuite est ensuite refroidie de la température de 50°C à 43°C en 15 heures dans un cristallisoir continu.

On obtient alors des eaux mères présentant, pour une matière sèche de 72 %, une composition de :
- 65 % sur sec de maltitol,
- 13 % sur sec de sorbitol,
- 18 % sur sec de DP ≥ 3 hydrogénés,
- 4 % de produits de cracking.

La composition cristalline obtenue contient quant à elle 98,1 % sur sec de maltitol, 0,1 % sur sec de sorbitol et 2,3 % sur sec de DP ≥ 3 hydrogénés.

Ces conditions de cristallisation permettent d'obtenir des formes cristallines qui sont toutes majoritairement de type parallélépipède rectangulaire telles qu'illustrées par les figures 2 à 5.

### Exemple 3

La cristallisation du sirop de maltitol D de l'exemple 1 est effectuée par refroidissement en deux étapes.

La première étape est une phase dite d'évaporation. Il s'agit de concentrer le sirop afin d'obtenir une solution présentant un degré de sursaturation compris entre 1,05 et 1,07.

La quantité d'eau à évaporer est déterminée par le calcul à partir de la concentration initiale.

Lorsque la sursaturation désirée est atteinte, l'ensemencement est réalisé par l'introduction d'une quantité de semence calibrée à 35 µm.

La période qui suit cet ensemencement est composée de plusieurs phases de refroidissement telles que détaillées dans le tableau I suivant.

**Tableau I**

| | Rampe de température | Variation de température | Durée | Vitesse pratique | Vitesse moyenne |
|---|---|---|---|---|---|
| ETAPE 1 | 80 à 64°C | 16°C | 48 h | 2°C / 6 h | 0,33°C/ h |
| ETAPE 2 | 64 à 52°C | 12°C | 18 h | 2°C / 3 h | 0,66°C / h |
| ETAPE 3 | 52 à 46°C | 6°C | 3 h | 2°C / 1 h | 2°C / h |
| Au total : | 80 à 46°C | 55°C | 80 h | 0,68°C / h | 0,68°C / h |

On obtient alors des eaux mères présentant, pour une matière sèche de 72 % :
- 67 % sur sec de maltitol
- 10 % sur sec de sorbitol,
- 21 % sur sec de DP ≥ 3 hydrogénés,
- 2 % de produits de cracking

Les formes cristallines obtenues sont toutes très majoritairement de type parallélépipède rectangulaire telles qu'illustrées par les figures 6 à 9, i.e. présentant plus particulièrement, en regard des formes obtenues dans le cadre de l'exemple 1 et illustrées par les figures 2 à 5, des troncatures au niveau de leurs deux plus petites faces.

Sans être liée par une quelconque théorie, la société Demanderesse attribue à la conduite de la cinétique de cristallisation ainsi décrite dans cet exemple l'apparition desdites troncatures sur les plus petites faces des formes parallélépipèdes rectangulaires conformes à l'invention ainsi observées.

### Exemple 4

Le tableau II suivant présente la caractérisation par diffraction X selon le test A des cristaux de maltitol selon l'invention et de poudres de maltitol présentant différentes formes cristallines (correspondant aux cristaux de la figure 1).

**Tableau II**

| | Forme parallélépipède rectangulaire selon l'invention | | | Forme parallélépipède plutôt cubique | | | Forme bipyramidale | | | Forme prismatique | | | Forme en aiguille | | |
|---|---|---|---|---|---|---|---|---|---|---|---|---|---|---|---|
| Intensité de la raie à 16,07° (%) | 0 | 0 | 0 | 0 | 0 | 0 | 0,14 | 0 | 0,1 | 0,26 | 0,21 | 0,14 | 0,42 | 0,42 | 0,33 |
| Intensité de la raie à 26,51° (%) | 100 | 100 | 100 | 54,46 | 60,87 | 81,76 | 67,3 | 60,1 | 73,7 | 100 | 100 | 100 | 100 | 100 | 100 |

Les intensités des raies à 16,07° et 26,51° permettent d'identifier et de différencier les cinq formes cristallines du maltitol cristallin.

## Revendications

1. Cristaux de maltitol **caractérisés par le fait qu'**ils sont de forme parallélépipède rectangulaire et que le rapport des dimensions longueur sur largeur est compris entre 1,8 et 5,3, de préférence compris entre 2,3 et 3,7.

2. Cristaux de maltitol selon la revendication 1, **caractérisés par le fait que** les deux plus petites faces du parallélépipède rectangulaire sont des parallélogrammes dont les côtés sont égaux.

3. Cristaux de maltitol selon la revendication 1, **caractérisés par le fait que** les deux plus petites faces du parallélépipède rectangulaire sont des parallélogrammes dont les côtés sont égaux deux à deux.

4. Cristaux de maltitol selon l'une quelconque des revendications 1 à 3, **caractérisés par le fait que** les deux plus petites faces du parallélépipède rectangulaire sont des parallélogrammes dont au moins un angle présente une troncature.

5. Cristaux de maltitol selon l'une quelconque des revendications 1 à 4, **caractérisés par le fait que** la dimension longueur du parallélépipède rectangulaire est comprise entre 50 et 750 µm, de préférence comprise entre 97 et 280 µm.

6. Cristaux de maltitol selon l'une quelconque des revendications 1 à 5, **caractérisé par le fait que** leur profil de diffraction X, déterminé selon un test A, présente pour la raie à 26,51°, une intensité de 100 %, et pour la raie à 16,07°, une intensité égale à 0 %.

7. Composition cristalline de maltitol, **caractérisé par le fait que** la fraction cristalline du maltitol est constituée d'au moins 50 % en poids de cristaux de forme parallélépipède rectangulaire selon l'une quelconque des revendications 1 à 6, de préférence de 70 à 100 % en poids, plus préférentiellement encore de 95 à 100 % en poids.

8. Procédé de préparation de cristaux de maltitol des revendications 1 à 6, **caractérisé par le fait que** l'on cristallise dans l'eau un sirop de maltitol en présence d'impuretés constituées d'aldéhydes et de composés dicarbonylés condensés.

9. Procédé selon la revendication 8, **caractérisé par le fait que** la quantité d'impuretés est inférieure à 5%, de préférence dans un intervalle de 1,5 à 4,5 %, et plus préférentiellement dans un intervalle de 2,5 à 3,5 %

10. Procédé selon les revendication 8 et 9, **caractérisé par le fait que** les aldéhydes et les composés dicarbonylés condensés sont obtenus par un traitement thermique à plus de 100°C de solutions glucidiques choisies dans le groupe constitué de saccharose, fructose, sucres invertis et sirops de glucose.

11. Procédé selon la revendication 10, **caractérisé par le fait que** l'on :
a) prépare un premier sirop de maltitol d'une matière sèche au moins égale à 60 % en poids et présentant une teneur en maltitol au moins égale à 85 % en poids,
b) introduit dans ce sirop de maltitol un sucre choisi dans le groupe constitué par le saccharose, le fructose, les sucres invertis, les sirops de glucose, seuls ou en mélange
c) chauffe ce premier sirop de maltitol de manière à générer des impuretés constituées d'aldéhydes et de composés dicarbonylés condensés,
d) prépare un deuxième sirop de maltitol d'une matière sèche au moins égale à 65 % en poids et présentant une teneur en maltitol au moins égale à 90 % en poids,
e) incorpore ce deuxième sirop de maltitol dans le premier sirop de maltitol de manière à obtenir un sirop de maltitol d'une matière sèche au moins égale à 70 % en poids et présentant une teneur en maltitol au moins égale à 80 % en poids,
f) concentre le sirop de maltitol ainsi obtenu à une matière sèche au moins égale à 80 %,
g) cristallise par évaporation et/ou par refroidissement le sirop de maltitol ainsi concentré,
h) récupère les cristaux ainsi obtenus.

## Patentansprüche

1. Maltitol-Kristalle, **dadurch gekennzeichnet, dass** sie in Form eines rechteckigen Parallelepipeds sind, und dass das Dimensionsverhältnis von Länge zu Breite zwischen 1,8 und 5,3 umfasst ist, vorzugsweise zwischen 2,3 und 3,7 umfasst ist.

2. Maltitol-Kristalle nach Anspruch 1, **dadurch gekennzeichnet, dass** die zwei kleineren Seiten des rechteckigen Parallelepipeds Parallelogramme sind, dessen Seiten gleich sind.

3. Maltitol-Kristalle nach Anspruch 1, **dadurch gekennzeichnet, dass** die zwei kleineren Seiten des rechteckigen Parallelepipeds Parallelogramme sind, dessen Seiten paarweise gleich sind.

4. Maltitol-Kristalle nach einem der Ansprüche 1 bis 3, **dadurch gekennzeichnet, dass** die zwei kleineren Seiten des rechteckigen Parallelepipeds Parallelogramme sind, von denen mindestens ein Winkel eine Abstumpfung aufweist.

5. Maltitol-Kristalle nach einem der Ansprüche 1 bis 4, **dadurch gekennzeichnet, dass** die Länge des rechteckigen Parallelepipeds zwischen 50 und 750 µm umfasst ist, vorzugsweise zwischen 97 und 280 µm umfasst ist.

6. Maltitol-Kristalle nach einem der Ansprüche 1 bis 5, **dadurch gekennzeichnet, dass** ihr Röntgenbeugungsprofil, bestimmt gemäß einem Test A, auf der Beugungslinie bei 26,51° eine Intensität von 100%, und auf der Beugungslinie bei 16,07° eine Intensität gleich 0% aufweist.

7. Kristalline Maltitol-Zusammensetzung, **dadurch gekennzeichnet, dass** die kristalline Maltitol-Fraktion aus mindestens 50 Gewichts-% von Kristallen in Form eines rechteckigen Parallelepipeds nach einem der Ansprüche 1 bis 6, vorzugsweise von 70 bis 100 Gewichts-%, am meisten bevorzugt von 95 bis 100 Gewichts-%, besteht.

8. Verfahren zur Herstellung von Maltitol-Kristallen der Ansprüche 1 bis 6, **dadurch gekennzeichnet, dass** ein Maltitolsirup im Wasser in Gegenwart von Verunreinigungen, die aus Aldehyden und kondensierten Dicarbonylverbindungen bestehen, kristallisiert wird.

9. Verfahren nach Anspruch 8, **dadurch gekennzeichnet, dass** die Menge an Verunreinigungen weniger als 5%, vorzugsweise in einem Bereich von 1,5 bis 4,5%, am meisten bevorzugt in einem Bereich von 2,5 bis 3,5% beträgt.

10. Verfahren nach den Ansprüchen 8 und 9, **dadurch gekennzeichnet, dass** die Aldehyde und die kondensierten Dicarbonylverbindungen erhalten werden durch eine Wärmebehandlung von Kohlenhydratlösungen ausgewählt aus der Gruppe, bestehend aus Saccharose, Fruktose, Invertzucker und Glukosesirupen bei mehr als 100 °C.

11. Verfahren nach Anspruch 10, **dadurch gekennzeichnet, dass**:
a) ein erster Maltitolsirup mit einer Trockenmasse mindestens gleich 60 Gewichts-%, und welcher einen Maltitol-Gehalt mindestens gleich 85 Gewichts-% aufweist, hergestellt wird,
b) ein Zucker ausgewählt aus der Gruppe bestehend aus Saccharose, Fruktose, Invertzucker und Glukosesirupen, allein oder gemischt, in den Maltitolsirup eingeführt wird,
c) dieser erste Maltitolsirup aufgewärmt wird, um Verunreinigungen, die aus Aldehyden und kondensierten Dicarbonylverbindungen bestehen, zu erzeugen,
d) ein zweiter Maltitolsirup mit einer Trockenmasse mindestens gleich 65 Gewichts-%, und welcher einen Maltitol-Gehalt mindestens gleich 90 Gewichts-% aufweist, hergestellt wird,
e) der zweite Maltitolsirup in den ersten Maltitolsirup eingebracht wird, um ein Maltitolsirup mit einer Trockenmasse mindestens gleich 70 Gewichts-%, und welcher einen Maltitol-Gehalt mindestens gleich 80 Gewichts-% aufweist, zu erhalten,
f) der so erhaltene Maltitolsirup bis zu einer Trockenmasse mindestens gleich 80 Gewichts-% konzentriert wird,
g) der so konzentrierte Maltitolsirup durch Verdunstung und/oder durch Kühlung kristallisiert wird,
h) die so erhaltenen Kristalle gewonnen werden.

## Claims

1. Maltitol crystals, **characterized in that** they have a rectangular parallelepipedal shape and **in that** the ratio of the length to width dimensions is in the range 1.8 to 5.3, preferably in the range 2.3 to 3.7.

2. Maltitol crystals according to Claim 1, **characterized in that** the two smallest faces of the rectangular parallelepiped are parallelograms with equal sides.

3. Maltitol crystals according to Claim 1, **characterized in that** the two smallest faces of the rectangular parallelepiped are parallelograms with paired equal sides.

4. Maltitol crystals according to any one of Claims 1 to 3, **characterized in that** the two smallest faces of the rectangular parallelepiped are parallelograms whereof at least one angle comprises a truncation.

5. Maltitol crystals according to any one of Claims 1 to 4, **characterized in that** the length dimension of the rectangular parallelepiped is in the range 50 to 750 µm, preferably in the range 97 to 280 µm.

6. Maltitol crystals according to any one of Claims 1 to 5, **characterized in that** their X-ray diffraction profile, determined using a test A, has an intensity of 100% for the peak at 26.51° and an intensity of 0% for the peak at 16.07°.

7. Crystalline maltitol composition, **characterized in that** the crystalline fraction of the maltitol is constituted by at least 50% by weight of crystals with a rectangular parallelepipedal shape in accordance with any one of Claims 1 to 6, preferably 70% to 100% by weight, more preferably 95% to 100% by weight.

8. Process for preparing the maltitol crystals of Claims 1 to 6, **characterized in that** a maltitol syrup is crystallized in water in the presence of impurities constituted by aldehydes and condensed dicarbonyl compounds.

9. Process according to Claim 8, **characterized in that** the quantity of impurities is less than 5%, preferably in the range 1.5% to 4.5%, and more preferably in the range 2.5% to 3.5%.

10. Process according to Claims 8 and 9, **characterized in that** the aldehydes and the condensed dicarbonyl compounds are obtained by a heat treatment at more than 100°C of glucidic solutions selected from the group constituting of saccharose, fructose, invert sugars and glucose syrups.

11. Process according to Claim 10, **characterized in that** it comprises:
a) preparing a first maltitol syrup with a dry matter content of at least 60% by weight and with a maltitol content of at least 85% by weight;
b) introducing into said maltitol syrup a sugar selected from the group constituting of saccharose, fructose, invert sugars, and glucose syrups, used alone or as a mixture;
c) heating said first maltitol syrup in order to generate impurities constituted by aldehydes and condensed dicarbonyl compounds;
d) preparing a second maltitol syrup with a dry matter content of at least 65% by weight and with a maltitol content of at least 90% by weight;
e) incorporating said second maltitol syrup into the first maltitol syrup in order to obtain a maltitol syrup with a dry matter content of at least 70% by weight and with a maltitol content of at least 80% by weight;
f) concentrating the maltitol syrup obtained to a dry matter content of at least 80%;
g) crystallizing the concentrated maltitol syrup by evaporation and/or cooling;
h) recovering the crystals thereby obtained.
